# FASCICULE DE BREVET EUROPEEN

(11) **EP 2 285 425 B1**
(45) Date de publication et mention de la délivrance du brevet: **03.05.2017**
(21) Numéro de dépôt: 09757703.5
(22) Date de dépôt: 05.06.2009
(51) Int. Cl.: A61L 2/18, C11D 3/04, A61L 101/22, C11D 3/39, C11D 3/48, A01N 59/20

(54) **COMPOSITION POUR LA DESINFECTION ET LA DECONTAMINATION DE CORPS CONTAMINES PAR DES PRIONS AINSI QUE PAR DES AGENTS TRANSMISSIBLES CONVENTIONNELS**
VERWENDUNG EINER ZUSAMMENSETZUNG ZUR DESINFEKTION UND DEKONTAMINIERUNG VON DURCH PRIOREN UND KONVENTIONELLE ÜBERTRAGBARE ERREGER KONTAMINIERTEN GEGENSTÄNDEN
USE OF A COMPOSITION FOR DISINFECTION AND DECONTAMINATION OF OBJECTS CONTAMINATED BY PRIONS AND CONVENTIONAL TRANSMISSIBLE AGENTS

(30) Priorité: 05.06.2008 FR 0803130
(43) Date de publication de la demande: 23.02.2011
(73) Titulaire: Laboratoires Anios, 59260 Lille-Hellemmes (FR)
(72) Inventeur: LETARTRE, Bertrand, F-59260 Lille Hellemmes (FR); TERRIER, Sébastien, F-59260 Lille Hellemmes (FR); RAUWEL, Gaëtan, F-59260 Lille Hellemmes (FR); CRIQUELION, Jacques, F-59260 Lille Hellemmes (FR)
(74) Mandataire: Boubal, Denis Henri Jacques
(86) Numéro de dépôt international: PCT/FR2009/000659
(87) Numéro de publication internationale: WO 2009/147327

(56) Documents cités:
- WO-A-2004/020562
- DE-A1- 19 639 603
- FR-A- 2 790 390
- FR-A- 2 870 458
- GB-A- 2 257 630
- US-A- 4 414 127
- US-A- 4 614 646
- US-A- 5 900 256

## Description

L'invention concerne l'utilisation d'une composition pour la désinfection et la décontamination simultanées d'objets ou surfaces contaminés par des agents transmissibles conventionnels (ATC) tels que notamment bactérie, levure, spore de moisissures, mycobactérie, virus, spore de bactérie, et des agents transmissibles non conventionnels (ATNC) tels que des prions.

L'invention trouvera notamment une application particulière pour le traitement d'objets thermosensibles tels que des endoscopes.

Lorsqu'un objet, à usages multiples, est suspecté d'être infecté par un agent transmissible conventionnel ou non conventionnel, il est recommandé soit :
- de le détruire par incinération si les données économiques le permettent, et dans le cas contraire,
- de le stériliser par vapeur (autoclavage) s'il est thermorésistant, sinon,
- de le traiter par un traitement dit de désinfection et/ou de décontamination.

L'invention ici décrite s'intéresse plus particulièrement à la troisième situation lorsque l'objet est notamment thermosensible et à usages multiples.

Un objet susceptible d'être contaminé par un ATC et/ou ATNC fait suite à une utilisation de cet objet au contact interne et/ou externe d'un organisme. On peut citer le cas de l'usage d'un matériel endoscopique lors d'un examen médical exploratoire. Au contact de certains organes et/ou fluides organiques humains ou animaux, l'objet peut être contaminé par des ATNC mais aussi, dans le même temps, par des ATC.

Il est recommandé entre les examens sur deux patients différents de procéder à des opérations qui visent à maîtriser le risque infectieux par ATNC et ATC.

A ce jour, il n'existe pas de procédure simple et efficace qui permette de, conjointement, désinfecter vis-à-vis des ATC et décontaminer vis-à-vis des ATNC, des objets à usages multiples thermosensibles et pH alcalins sensibles.

La procédure de désinfection des objets thermosensibles à usages multiples par immersion est connue et pratiquée. La procédure de décontamination vis-à-vis des ATNC d'objets, par immersion, par des procédures de pH alcalin à très alcalin (solution d'hydroxyde de sodium ou d'hydroxyde de potassium ou de polyéthanolamine ayant des pH allant de 10 à 14) est, de même, connue. A ce jour, il n'est pas mis en oeuvre de procédure par immersion permettant de décontaminer vis-à-vis des ATNC, des objets sensibles au pH alcalin.

On connaît toutefois du document WO 2005/118762 le fait d'utiliser le cuivre et ses dérivés, pour la décontamination de produits contaminés par des prions. Le traitement de décontamination est réalisé de préférence avec une solution renfermant ledit composé à raison d'au moins 500 micromoles de dérivés métalliques. Cette solution peut contenir du peroxyde d'hydrogène (H₂O₂) dans la solution à raison d'environ 50 millimoles.

Une telle composition permet ainsi de décontaminer des corps souillés par des prions mais ne permet pas, en raison de la faible proportion en H₂O₂, de désinfecter des ATC tels que notamment bactérie, levure, virus ou autres, avec une efficacité validée selon les standards européens (e.g normes EN 14561, EN 14562, EN 14563, EN 14476...).

Il est particulièrement difficile d'identifier, a priori, le risque de contamination par ATNC. Seuls des signes pathologiques apparents ou un historique renseignant du profil médical d'un individu peut permettre d'appréhender le risque. Une décontamination systématique de tout objet susceptible d'être contaminé par des ATNC est souhaitable afin de limiter tout risque de contamination croisée.

Afin de faciliter la prise en charge d'une opération de décontamination et de la rendre opérable avec les procédures de nettoyage et de désinfection des objets, la présente invention a pour objet d'obtenir une décontamination vis-à-vis des ATNC en même temps que les opérations courantes de nettoyage et de désinfection.

Plus particulièrement, le but de la présente invention est de proposer une composition pour la désinfection et la décontamination simultanées d'objets ou surfaces contaminés par des agents transmissibles conventionnels (ATC) et des agents transmissibles non conventionnels (ATNC) tels que des prions.

Un autre but de l'invention est de proposer une telle composition particulièrement adaptée pour un traitement manuel, notamment par trempage.

Un autre but de l'invention est de proposer un procédé de préparation d'une telle composition à partir de deux solutions, respectivement à base de cuivre et à base de peroxyde d'hydrogène.

Un autre but de l'invention est de proposer une solution aqueuse de cuivre permettant la préparation rapide d'une composition conforme à l'invention, en mélange avec une solution aqueuse de peroxyde d'hydrogène.

Un autre but de la présente invention est de proposer une solution aqueuse de peroxyde d'hydrogène destinée à être mélangée à une solution aqueuse de cuivre pour la réalisation d'une composition conforme à l'invention.

D'autres buts et avantages de la présente invention apparaîtront au cours de la description suivante qui n'est donnée qu'à titre indicatif et qui n'a pas pour but de la limiter.

L'invention concerne tout d'abord l'utilisation d'une composition pour la désinfection et/ou la décontamination simultanées d'objets ou surfaces contaminés par des prions et des agents transmissibles conventionnels (ATC), tels que notamment bactérie, levure, spore de moisissure, mycobactérie, spore de bactérie, virus et notamment pour le traitement d'objets thermosensibles tels que des endoscopes.

Selon l'invention, la dite composition comprend une solution de peroxyde d'hydrogène et une solution aqueuse de cuivre ou un de ses dérivés tel que sel de cuivre, ainsi que d'au moins un composé tensioactif anionique salifié inhibiteur de la décomposition du peroxyde d'hydrogène en présence de cuivre, la concentration en peroxyde d'hydrogène étant comprise entre 5% et 9% en poids.

La description concerne également une solution aqueuse de cuivre destinée à être mélangée à une solution aqueuse de peroxyde d'hydrogène pour la réalisation de la composition conforme à l'invention.

La solution aqueuse de cuivre utilisée conformément à l'invention comprend au moins un composé tensioactif anionique salifié inhibiteur de la décomposition du peroxyde d'hydrogène en présence de cuivre.

La description concerne également une solution aqueuse de peroxyde d'hydrogène destinée à être mélangée à une solution aqueuse de cuivre pour la réalisation de la composition conforme à l'invention.

La solution aqueuse de peroxyde d'hydrogène utilisée conformément à l'invention contient des agents stabilisants de peroxyde d'hydrogène choisis parmi les phosphonates, les perborates, d'une teneur comprise entre 0,002 % et 0,07 % en poids.

La description concerne également un procédé de préparation de la composition mise en oeuvre conformément à l'invention dans lequel on mélange extemporanément une solution aqueuse de cuivre et une solution aqueuse de peroxyde d'hydrogène.

L'invention concerne l'application de la composition conforme à l'invention et/ou de la solution aqueuse de cuivre en mélange avec une solution peroxydes d'hydrogène pour la désinfection et la décontamination simultanées d'objets ou surfaces contaminés par des ATC tels que notamment bactérie, levure ou autres et des prions.

L'invention sera mieux comprise à la lecture de la description suivante.

L'activité de certains métaux notamment du cuivre, utilisé sous forme de sel, a été démontrée pour inactiver avec efficacité les agents pathogènes de type agents transmissibles non conventionnels. L'efficacité vis-à-vis de ces agents peut être améliorée en présence de peroxyde, notamment de peroxyde d'hydrogène. Malheureusement, ces deux composés sont de caractère très attractif l'un vis-à-vis de l'autre : le sel de cuivre, tels que sulfate de cuivre, est un composé réducteur, tandis que le peroxyde d'hydrogène est un oxydant puissant.

Les peroxydes sont actifs sur les ATC en concentration élevée pour satisfaire au standard d'évaluation des activités biocides pour des temps acceptables en pratique médicale ou industrielle. Les concentrations en peroxyde d'hydrogène peuvent être de 1 à 10 % selon le temps d'action souhaité. Dans le cas de ce procédé et pour être en adéquation avec les pratiques dans le domaine de la santé ou industriel qui requièrent des temps courts de 10 à 30 minutes de traitement, la concentration la plus pertinente en peroxyde d'hydrogène peut être environ de 6,5 à 8,5 %. Ces fortes concentrations en oxydant renforcent singulièrement l'incompatibilité oxydant / réducteur de l'association peroxyde d'hydrogène / sel de cuivre.

L'invention vise donc plus particulièrement à rendre compatibles et utilisables ces composés réducteurs et oxydants par nature incompatibles entre eux, afin de maintenir les efficacités couplées de désinfection et de décontamination vis-à-vis des ATNC et ATC.

Le mélange simple de sel de cuivre et de peroxyde d'hydrogène entraîne une décomposition quasi instantanée des peroxydes avec dégagement d'oxygène. Afin de proposer un procédé utilisable disposant au moins d'une durée minimale de stabilité et donc de vente, il conviendra notamment d'isoler ces deux composés dans deux récipients distincts. Cette seule approche nécessaire n'est toutefois pas suffisante car l'objectif est de garantir une stabilité, donc un usage de la solution après mélange des récipients contenant respectivement la solution aqueuse de cuivre et la solution de peroxyde d'hydrogène.

La reconstitution par simple mélange entraînera ladite décomposition qui s'initie instantanément et aboutit à une dégradation partielle à totale du peroxyde d'hydrogène : la conséquence de ce mélange aboutit à une perte d'efficacité partielle à totale vis-à-vis des ATNC et des ATC comme décrit dans le tableau 1 :

**Tableau 1 : stabilité du peroxyde d'hydrogène en présence uniquement de sulfate de cuivre.**

| | T0 | T = 15 min | T = 24 heures | T = 72 heures |
|---|---|---|---|---|
| Concentration H₂O₂ (poids) | 7,5% | 7,4% | 2,62% | 0,37% |

**Tableau 1 bis : activités comparées du peroxyde d'hydrogène, des sels de cuivre et du mélange selon l'invention suivant la norme EN 13727 vis-à-vis de Staphylococcus aureus.**

| | H₂O₂ 7,5% | CuSO₄ (0,5 millimoles) | Mélange de l'invention | | | |
|---|---|---|---|---|---|---|
| | | | T=30 min | T = 24 h | T = 72 h | T = 7 jours |
| Staphylococcus aureus | Actif > 5 log | Inefficace | Actif > 5 log | Actif > 5 log | inefficace | inefficace |

L'utilisation de composants salifiés par la soude ou la potasse parmi lesquels l'alkybenzenesulfonate de sodium, l'alkylethersulfate de sodium, l'alkylsulfate de sodium, interfère la réaction de décomposition du peroxyde d'hydrogène par les sels de cuivre. Comme démontré dans le tableau 2, la quantité et l'identité sont primordiales pour gérer l'interaction entre les deux composants. L'alkylsulfate et l'alkylethersulfate de sodium sont notamment deux sels présentant de bonnes performances. La chaîne alkyl en C12 a particulièrement été évaluée.

**Tableau 2 : stabilité du peroxyde d'hydrogène en présence de sels de cuivre en fonction de la concentration (% poids) en composant anionique.**

| Données à +/-0.1% | Concentration en anioniques | T0 | T=15 min | T = 24 heures | T = 72 heures |
|---|---|---|---|---|---|
| Alkylethersulfate de sodium | 0,1% | 7,6% | 7,6% | 7,3% | 6,9% |
| | 1% | 7,6% | 7,6% | 7,6% | 7,6% |
| Alkyl benzène sulfonate de sodium | 0,1% | 7,6% | 7,6% | 7,4% | 7,3% |
| | 1% | 7,5% | 7,5% | 7,5% | 7,5% |
| Alkyl (dodecyl) sulfate de sodium | 0,1% | 7,6% | 7,6% | 7,6% | 7,4% |
| | 1% | 7,6% | 7,6% | 7,6% | 7,6% |

L'invention concerne ainsi l'utilisation d'une composition pour la désinfection et la décontamination simultanées de corps contaminés par des agents transmissibles conventionnels (ATC), tels que notamment bactérie, levure, spore de moisissure, mycobactérie, virus, spore de bactérie, et des prions, notamment pour le traitement d'objets thermosensibles tels que des endoscopes.

Par corps, on entend ici tout objet, notamment à usages multiples, et thermosensibles, ou encore toute surface telle que notamment sol ou paillasse.

Selon l'invention, la composition comprend une solution de peroxyde d'hydrogène et une solution aqueuse de cuivre ou un de ses dérivés tel que sel de cuivre, ainsi que d'au moins un composé tensioactif anionique salifié inhibiteur de la décomposition du peroxyde d'hydrogène en présence du cuivre.

Selon un mode de réalisation, la composition peut présenter (% en poids) :
- sels de cuivre anhydre entre 0,005% et 0,01%,
- peroxyde d'hydrogène entre 5% et 9 %,
- composé tensioactif anionique salifié entre 0,5% et 1,5 %,
- eau déminéralisée q.s 100%
la somme des composants étant égale à 100 %.

Afin de conférer des propriétés antimicrobiennes sans altérer les propriétés prionicides de la composition, la concentration en peroxyde d'hydrogène est comprise entre 5% et 9% en poids.

Ledit au moins un composé tensioactif anionique salifié peut être choisi parmi le groupe (non limitatif) suivant : dodecylbenzenesulfonate, dodecylsulfate, dodecylethersulfate de sodium.

La pureté du sel anionique est importante tel qu'illustré au tableau 3 pour la stabilité de l'agent oxydant.

**Tableau 3 : stabilité du peroxyde d'hydrogène en présence de sels de cuivre en fonction de la pureté du sel anionique**

| | T0 | T = 15 min | T = 24 heures |
|---|---|---|---|
| Qualité industrielle | 7,5% (+/- 0.1) | 7,3% (+/- 0.1) | 7,2% (+/- 0.1) |
| Haut niveau de pureté (synthèse) | 7,5% (+/- 0.1) | 7,5% (+/- 0.1) | 7,5% (+/- 0.1) |

Ledit au moins un composé anionique salifié doit être ainsi avantageusement ultra pur, de qualité dénommée de synthèse.

L'instabilité des sels de cuivre peut se manifester par des dépôts à l'interface air/solution du mélange de la solution de cuivre et de la solution de peroxyde d'hydrogène. Si ces dépôts sont initiés, la présence de sels, notamment de calcium et de magnésium, dans l'eau de rinçage (eau de réseau) des objets après nettoyage et avant trempage dans la composition en augmente la quantité ce qui peut se révéler pénalisant lors du traitement d'objets avec des canaux de petit diamètre.

La présence de sels de cuivre peut nécessiter d'optimiser leur solubilité, notamment avec les oxydants. L'insolubilité ou la solubilité partielle des sels de cuivre est renforcée par les sels de calcium et de magnésium, entre autres amenés par l'eau de rinçage des objets après nettoyage et avant trempage dans la composition. Les composants de type alkyl poly glucoside en quantité et en qualité, peuvent être particulièrement efficaces notamment en les associant en proportion choisie selon le nombre de carbone des chaînes alkyles. Selon un mode de réalisation, la composition peut aussi comprendre ainsi des tensioactifs de type alcool gras poly glucosides spécialement choisis, tels que notamment les alkyl en C4, afin de solubiliser les sels de cuivre.

L'utilisation dans la solution de cuivre de quantités importantes de sels anioniques, d'alkylpolyglucosides dans le but de solubiliser les sels de cuivre en présence notamment de sels de calcium, de magnésium ou autres, induit un phénomène d'instabilité et de déphasage par surconcentration. Les composés, notamment les agents hydrotropes, notamment, de type toluène sulfonate de sodium ou xylène sulfonate sont connus pour être des composés d'élection pour résoudre ces situations.

**Tableau 4 : présence de dépôt selon la présence de composés de type alkylpolyglucosides**

| | T0 | T = 24 heures | T = 7 jours |
|---|---|---|---|
| Solution de H₂O₂ + sels de Cu +dérivés anioniques | non | oui | oui |
| Solution de H₂O₂ + sels de Cu + dérivés anioniques + alkylpolyglucosides | non | non | non |

L'adjonction de composés de type alcolyxat d'alcool gras choisis selon leur balance hydrophile/lipophile, en association avec des tiers solvants de type alcool à nombre de carbones notamment compris entre 4 et 6 favorables à ce mélange complexe, peut conférer le meilleur équilibre qui assure une stabilité dans le temps, notamment aux températures basses : plus de 1 an pour des températures allant de + 5 °C à + 35 °C. La composition peut présenter des tensioactifs alcools gras poly alcoxylés aptes à assurer un caractère hydrotrope en association avec un tiers solvant de type alcool ou polyol, favorisant notamment la stabilité au froid.

L'utilisation d'un agent colorant peut être nécessaire pour pouvoir distinguer la composition d'une autre solution qui coexisterait sur un même plan de travail. Le choix du colorant peut être spécifiquement étudié car il doit coexister pendant un certain temps, notamment plusieurs jours dans la composition, notamment après mélange d'une solution à base de cuivre et d'une solution de peroxyde d'hydroxyde dont les agents oxydants sont par ailleurs connus pour leur qualité de décolorant. Un colorant bleu, de triarylméthane peut présenter un bon compromis : il colore la solution avec une stabilité satisfaisante dans le temps, sans pour autant colorer les objets à traiter.

Selon un mode de réalisation, la composition peut comprendre (% en poids) :
- sels de cuivre entre 0,005% et 0,01 %,
- peroxyde d'hydrogène entre 5% et 9%,
- composé tensioactif anionique salifié entre 0,5% et 1,5%,
- tensioactifs de type alcool gras polyglucoside entre 0,1% et 0,4%,
- tensioactif alcool gras polyalcoxylé entre 0,1% et 0,3%,
- tiers solvant de type alcools ou polyols, entre 0,1 % et 0,3%,
- colorant q.s,
- eau déminéralisée, q.s. 100 %,
la somme des composants étant égale à 100 %.

L'invention concerne également un procédé de préparation de la composition conforme à l'invention dans lequel on mélange extemporanément une solution aqueuse de cuivre et une solution aqueuse de peroxyde d'hydrogène.

Ladite solution aqueuse de cuivre peut être ajoutée à la solution de peroxydes dans les proportions indicatives respectives de 1/25.

La solution aqueuse de cuivre destinée à être mélangée à une solution aqueuse de peroxyde peut, selon un mode de réalisation, comprendre en outre au moins un composé tensioactif anionique salifié inhibiteur de la décomposition du peroxyde d'hydrogène en présence de cuivre.

Le composé tensioactif anionique salifié peut être choisi parmi le groupe suivant : dodecylbenzenesulfonate, dodecylsulfate, dodecylethersulfate de sodium, d'une teneur comprise entre 1 et 50 %, notamment entre 20 et 30 %.

Selon un exemple de réalisation, le composé tensioactif est du dodecylsulfate ou dodecylethersulfate de sodium d'une teneur comprise entre 20 et 30 % en poids, permettant de donner notamment une concentration finale dans la composition conforme à l'invention d'environ 1 %.

Ledit au moins un composé anionique salifié peut être ultra pur de qualité dénommée de synthèse, notamment pauvre en impuretés, et autres résidus de synthèse, aptes à catalyser la dégradation, entre autres des peroxydes.

La solution aqueuse de cuivre peut comprendre au moins un tensioactif de type alcool gras poly glucoside afin de solubiliser les sels de cuivre, d'une teneur comprise entre 2,5 et 10 % en poids, pour donner notamment une concentration finale dans la composition conforme à l'invention d'environ à 0,1 à 0,4 %.

La solution aqueuse de cuivre peut comprendre au moins un tensioactif de type alcool gras poly alcoxylé afin de stabiliser la composition, d'une teneur comprise entre 2,5 et 7,5 % en poids, pour donner notamment une concentration finale dans la composition utilisée conformément à l'invention d'environ 0,1 à 0,3 %.

La solution aqueuse de cuivre peut présenter une teneur de sels de cuivre comprise entre 0,1% et 0,25 % en poids exprimé en sulfate, permettant notamment de donner une concentration finale dans la solution reconstituée de 0,005% à 0,01 %.

La solution aqueuse de cuivre peut comprendre un tiers solvant de type alcool ou polyols d'une teneur comprise entre 2 et 10 %, notamment entre 4 et 7 %.

La solution aqueuse de cuivre peut comprendre un colorant, notamment bleu, de type triarylméthane, compatible avec les sels métalliques et le peroxyde d'hydrogène.

La solution aqueuse de cuivre peut comprendre (pourcentage en poids) :
- sels de cuivre entre 0,1% et 0,25 %
- composé tensioactif anionique salifié entre 20% et 30%,
- tensioactifs de type alcool gras polyglucoside entre 2,5% et 10%,
- tensioactif alcool gras polyalcoxylé 2,5% et 7,5%,
- tiers-solvant de type alcools gras ou polyols entre 4% et 7%,
- colorant, q.s,
- eau déminéralisée q.s 100 %,
la somme des composants étant égale à 100 %.

Cette solution de cuivre peut être ajoutée à la solution de peroxydes dans les proportions indicatives respectives de 1/25.

La description concerne également une solution aqueuse de peroxyde d'hydrogène destinée à être mélangée à une solution aqueuse de cuivre, notamment telle que précédemment décrite pour la réalisation d'une composition utilisée conformément à l'invention.

Les solutions d'oxydants sont particulièrement fragilisées par la présence de sels de métaux. Aussi est-il préférable de protéger ces solutions oxydantes, d'une part, par un choix de haute qualité analytique, et d'autre part, par l'adjonction de phosphonates, persels de sodium ou de potassium. L'ajout peut être en quantité juste suffisante pour inerter tout composant minéral ou métallique indésirable à la solution aqueuse de peroxyde d'hydrogène seule, ou en mélange avec la solution aqueuse à base de cuivre.

Selon l'invention, la solution de peroxyde d'hydrogène contient des agents stabilisants de peroxyde d'hydrogène choisis parmi les phosphonates, les perborates d'une teneur comprise entre 0,002 % et 0,07 % en poids.

Comme démontré dans le tableau 5, l'activité sur plusieurs microorganismes, selon la norme européenne EN 13727 et EN 13624, est supérieure pour une composition selon l'invention par rapport à une solution de peroxyde d'hydrogène et de sulfate de cuivre uniquement.

**Tableau 5 : activité comparée 15 minutes après le mélange selon les normes EN 13727 (réduction de la population microbienne de 5 log en 5 minutes) et EN 13624 (réduction de la population microbienne de 4 log en 15 minutes) à + 20°C**

| | Pseudomonas aeruginosa EN 13727 | | Candida albicans En 13624 | |
|---|---|---|---|---|
| | % Solution aqueuse de cuivre (en volume) | Réduction activité | % Solution aqueuse de cuivre (en volume) | Réduction activité |
| Solution de peroxyde d'hydrogène + solution sulfate de cuivre uniquement | 20% | < 4 log | 20% | <2.7log |
| | 40% | > 5.4log | 40% | > 4 log |
| Composition selon l'invention | 20% | > 5.4log | 20% | > 4 log |
| | 40% | > 5.4log | 40% | > 4 log |

L'influence de chaque composant de la solution de cuivre ainsi que de la solution de peroxyde d'hydrogène a été appréciée et validée vis-à-vis de l'activité sur les ATNC. Chaque étape de formulation a fait l'objet de mesures de performance selon la méthode de Western Blot.

Dans une procédure globale de traitement des objets, les performances de la composition ont été évaluées in vitro vis-à-vis des ATNC selon les techniques connues (techniques de Western Blot) sur au moins six souches d'origines diverses, et in vivo sur des animaux contaminés par un inoculum de cerveau infectieux.

La composition utilisée conformément à l'invention notamment constituée à partir d'une solution de peroxyde d'hydrogène mélangée avec une solution aqueuse de cuivre, selon l'invention, trouve ainsi une application pour la désinfection et la décontamination simultanées d'objets ou surfaces contaminés par des ATC, bactérie, levure, spores, virus et autres et des prions. Le procédé peut avantageusement présenter une étape préalable de détergence et/ou de désinfection pour éliminer une partie physiquement et/ou enzymatiquement des particules ATC ou ATNC.

Plus précisément, la procédure peut ainsi comprendre en première intention, l'usage d'un détergent, et/ou détergent désinfectant enzymatique ou non, apte à éliminer une partie non négligeable de matériel infectieux et quantifiée selon les méthodes in vitro et in vivo ci-dessus décrites. Dans un deuxième temps, on reproduit la première opération dans un temps plus ou moins équivalent puis, dans un troisième temps, on utilise la composition conforme à l'invention. Cette procédure assure dans le même temps une efficacité vis-à-vis des ATNC et une efficacité vis-à-vis des ATC validés selon les méthodes en vigueur et reconnues de mesures de performances des désinfectants.

Naturellement, d'autres modes de réalisation auraient pu être envisagés par l'homme de l'art sans pour autant sortir du cadre de l'invention définie par les revendications ci-après.

## Revendications

1. Utilisation d'une composition pour la désinfection et la décontamination simultanées d'objets ou surfaces contaminés par des prions et des agents transmissibles conventionnels (ATC), tels que notamment bactérie, levure, virus, spore de moisissure, mycobactérie, spore de bactérie, ladite composition comprenant une solution de peroxyde d'hydrogène et une solution aqueuse de cuivre ou un de ses dérivés tels que sels de cuivre, **caractérisé en ce qu'**elle comprend en outre au moins un composé tensioactif anionique salifié inhibiteur de la décomposition du peroxyde d'hydrogène en présence de cuivre et **en ce que** la concentration en peroxyde d'hydrogène dans la composition est comprise entre 5% et 9% en poids.

2. Utilisation selon la revendication 1 dans laquelle ladite composition comprend (% en poids):
- sels de cuivre anhydre entre 0,005% et 0,01 %,
- peroxyde d'hydrogène entre 5% et 9 %,
- composé tensioactif anionique salifié entre 0,5% et 1,5 %,
- eau déminéralisée q.s 100%,
la somme des composants étant égale à 100 %.

3. Utilisation selon la revendication 1 ou 2, dans laquelle ledit au moins un composé tensioactif anionique salifié est choisi parmi le groupe suivant: dodecylbenzenesulfonate, dodecylsulfate, dodecylethersulfate de sodium.

4. Utilisation selon l'une des revendications 1 à 3 dans laquelle ledit au moins un composé anionique salifié est ultra pur, de qualité de synthèse.

5. Utilisation selon l'une des revendications 1 à 4 dans laquelle la composition comprend des tensioactifs de type alcools gras polyglucoside spécialement choisis, notamment les alkyl en C4, afin de solubiliser les sels de cuivre.

6. Utilisation selon la revendication 5 dans laquelle ladite composition comprend des tensioactifs alcools gras polyalcoxylés aptes à assurer un caractère hydrotrope, en association avec un tiers-solvant de type alcools ou polyols.

7. Utilisation selon l'une des revendications 1 à 6, dans laquelle ladite composition comprend un colorant compatible avec les sels métalliques et le peroxyde d'hydrogène.

8. Utilisation selon la revendication 1, 3 ou 4, dans laquelle ladite composition comprend (% en poids):
- sels de cuivre entre 0,005% et 0,01 %,
- peroxyde d'hydrogène entre 5% et 9%,
- composé tensioactif anionique salifié entre 0,5% et 1 ,5%,
- tensioactifs de type alcool gras polyglucoside entre 0,1% et 0,4%,
- tensioactif alcool gras polyalcoxylé entre 0,1 % et 0,3%,
- tiers solvant de type alcools ou polyols, entre 0,1% et 0,3%,
- colorant q. s,
- eau, q.s. 100 %,
la somme des composants étant égale à 100 %.

9. Utilisation selon l'une des revendications 1 à 8 dans laquelle on prépare ladite composition par le mélange extemporané d'une solution aqueuse de cuivre et d'une solution aqueuse de peroxyde d'hydrogène.

10. Utilisation selon la revendication 9, dans laquelle ladite solution aqueuse de cuivre, destinée à être mélangée à une solution aqueuse de peroxyde d'hydrogène, comprend, en outre, ledit au moins un composé tensioactif anionique salifié inhibiteur de la décomposition du peroxyde d'hydrogène en présence de cuivre.

11. Utilisation selon la revendication 10 dans laquelle ledit composé tensioactif anionique salifié a une teneur comprise entre 1% et 50% (en poids) dans ladite solution aqueuse de cuivre.

12. Utilisation selon la revendication 10 ou 11 dans laquelle ladite solution aqueuse de cuivre présente du sel de cuivre à teneur comprise entre 0,1% et 0,25% (en poids).

13. Utilisation selon l'une des revendications 10 à 12, dans laquelle ledit au moins un composé tensioactif anionique salifié de la solution aqueuse de cuivre est choisi parmi le groupe suivant : dodecylbenzenesulfonate, dodecylsulfate, dodecylethersulfate de sodium.

14. Utilisation selon l'une des revendications 10 à 13, dans laquelle ledit au moins un composé anionique salifié de la solution aqueuse de cuivre est ultra pur, de qualité de synthèse.

15. Utilisation selon l'une des revendications 10 à 14, dans laquelle la solution aqueuse de cuivre comprend au moins un tensioactif de type alcools gras polyglucoside afin de solubiliser les sels de cuivre, d'une teneur comprise entre 2,5 et 10 % (% en poids).

16. Utilisation selon l'une des revendications 10 à 15, dans laquelle la solution aqueuse de cuivre comprend des tensioactifs alcools gras polyalcoxylé aptes à assurer un caractère hydrotrope, de teneur comprise entre 2,5% et 7,5% (% en poids).

17. Utilisation selon la revendication 16 dans laquelle la solution aqueuse de cuivre comprend un tiers-solvant de type alcools ou polyols d'une teneur comprise entre 4% et 7% (% en poids).

18. Utilisation selon l'une des revendications 10 à 17 dans laquelle la solution aqueuse de cuivre comprend un colorant compatible avec les sels métalliques et le peroxyde d'hydrogène.

19. Utilisation selon la revendication 10 dans laquelle la solution aqueuse de cuivre comprend (% en poids):
- sels de cuivre entre 0,1 % et 0,25 %
- composé tensioactif anionique salifié entre 20% et 30%,
- tensioactifs de type alcool gras polyglucoside entre 2,5% et 10%,
- tensioactif alcool gras polyalcoxylé 2,5% et 7,5%,
- tiers-solvant de type alcools gras ou polyols entre 4% et 7%,
- colorant, q.s,
- eau déminéralisée q.s 100 %,
la somme des composants étant égale à 100 %.

20. Utilisation selon l'une des revendications 9 à 19 dans laquelle ladite solution aqueuse de peroxyde d'hydrogène destinée à être mélangée à une solution aqueuse de cuivre, pour la réalisation de ladite composition contient des agents stabilisants de peroxyde d'hydrogène choisis parmi les phosphonates, les perborates d'une teneur comprise entre 0,002 % et 0,07 % en poids.

21. Utilisation selon l'une quelconque des revendications 9 à 20 dans laquelle la solution aqueuse de cuivre est ajoutée à la solution aqueuse de peroxyde d'hydrogène dans les proportions indicatives de 1/25.

22. Utilisation selon l'une quelconque des revendications 1 à 21 pour le traitement d'endoscopes.

23. Utilisation selon l'une quelconque des revendications 1 à 22, dans lequel on réalise préalablement des étapes de détergence et/ou de désinfection pour éliminer une partie, physiquement et/ou enzymatiquement des particules ATC ou prions.

## Patentansprüche

1. Verwendung einer Zusammensetzung zur gleichzeitigen Desinfektion und Dekontamination von Objekten oder Oberflächen, die durch Prione und konventionelle übertragbare Erreger (CTA), wie insbesondere Bakterien, Hefe, Viren, Schimmelsporen, Mycobakterien, Bakteriensporen, kontaminiert sind, wobei die Zusammensetzung eine Lösung von Wasserstoffperoxid und eine wässerige Lösung von Kupfer oder einem seiner Derivate, wie Kupfersalzen, umfasst, **dadurch gekennzeichnet, dass** diese außerdem mindestens eine versalzte anionische grenzflächenaktive Verbindung als Inhibitor der Zersetzung des Wasserstoffperoxids in Anwesenheit von Kupfer umfasst, und dass die Konzentration von Wasserstoffperoxid in der Zusammensetzung zwischen 5 Gew.-% und 9 Gew.-% beträgt.

2. Verwendung nach Anspruch 1, wobei die Zusammensetzung umfasst (Gew.-%):
- Salze von wasserfreiem Kupfer zwischen 0,005 % und 0,01 %,
- Wasserstoffperoxid zwischen 5 % und 9 %,
- versalzte anionische grenzflächenaktive Verbindung zwischen 0,5 % und 1,5 %,
- entmineralisiertes Wasser q.s. 100 %,
wobei die Summe der Bestandteile gleich 100 % ist.

3. Verwendung nach Anspruch 1 oder 2, wobei die mindestens eine versalzte anionische grenzflächenaktive Verbindung aus der folgenden Gruppe ausgewählt wird: Dodecylbenzolsulfonat, Dodecylsulfat, Dodecylethersulfat von Natrium.

4. Verwendung nach einem der Ansprüche 1 bis 3, wobei die mindestens eine versalzte anionische Verbindung ultrarein mit Synthesequalität ist.

5. Verwendung nach einem der Ansprüche 1 bis 4, wobei die Zusammensetzung speziell ausgewählte grenzflächenaktive Stoffe vom Polyglucosid-Fettalkohol-Typ, insbesondere C₄-Alkyle, umfasst, um die Kupfersalze zu solubilisieren.

6. Verwendung nach Anspruch 5, wobei die Zusammensetzung grenzflächenaktive polyalkoxylierte Fettalkohole, die geeignet sind, einen hydrotropen Charakter zu gewährleisten, in Assoziation mit einem Dritt-Lösungsmittel vom Alkohol- oder Polyol-Typ umfasst.

7. Verwendung nach einem der Ansprüche 1 bis 6, wobei die Zusammensetzung ein Färbemittel umfasst, das mit den Metallsalzen und dem Wasserstoffperoxid kompatibel ist.

8. Verwendung nach Anspruch 1, 3 oder 4, wobei die Zusammensetzung umfasst (Gew.-%):
- Kupfersalze zwischen 0,005 % und 0,01 %,
- Wasserstoffperoxid zwischen 5 % und 9 %,
- versalzte anionische grenzflächenaktive Verbindung zwischen 0,5 % und 1,5 %,
- grenzflächenaktive Stoffe vom Polyglucosid-Fettalkohol-Typ zwischen 0,1 % und 0,4 %,
- grenzflächenaktiver polyalkoxylierter Fettalkohol zwischen 0,1 % und 0,3 %,
- Dritt-Lösungsmittel vom Alkohol- oder Polyol-Typ zwischen 0,1 % und 0,3 %,
- Färbemittel q.s.,
- Wasser q.s. 100 %,
wobei die Summe der Bestandteile gleich 100 % ist.

9. Verwendung nach einem der Ansprüche 1 bis 6, wobei die Zusammensetzung durch unmittelbares Mischen einer wässerigen Kupfer-Lösung und einer wässerigen Wasserstoffperoxid-Lösung hergestellt wird.

10. Verwendung nach Anspruch 9, wobei die wässerige Kupfer-Lösung, die dazu bestimmt ist, mit einer wässerigen Wasserstoffperoxid-Lösung gemischt zu werden, außerdem die mindestens eine versalzte anionische grenzflächenaktive Verbindung als Inhibitor der Zersetzung des Wasserstoffperoxids in Anwesenheit von Kupfer umfasst.

11. Verwendung nach Anspruch 10, wobei die versalzte anionische grenzflächenaktive Verbindung einen Gehalt zwischen 1 % und 50 % (bezogen auf das Gewicht) in der wässerigen Kupfer-Lösung aufweist.

12. Verwendung nach Anspruch 10 oder 11, wobei die wässerige Kupfer-Lösung ein Kupfersalz mit einem Gehalt zwischen 0,1 % und 0,25 % (bezogen auf das Gewicht) aufweist.

13. Verwendung nach einem der Ansprüche 10 bis 12, wobei die mindestens eine versalzte anionische grenzflächenaktive Verbindung der wässerigen Kupfer-Lösung aus der folgenden Gruppe ausgewählt wird: Dodecylbenzolsulfonat, Dodecylsulfat, Dodecylethersulfat von Natrium.

14. Verwendung nach einem der Ansprüche 10 bis 13, wobei die mindestens eine versalzte anionische Verbindung der wässerigen Kupfer-Lösung ultrarein mit Synthesequalität ist.

15. Verwendung nach einem der Ansprüche 10 bis 14, wobei die wässerige Kupfer-Lösung mindestens einen grenzflächenaktiven Stoff vom Polyglucosid-Fettalkohol-Typ, um die Kupfersalze zu solubilisieren, mit einem Gehalt zwischen 2,5 % und 10 % (Gew.-%) umfasst.

16. Verwendung nach einem der Ansprüche 10 bis 15, wobei die wässerige Kupfer-Lösung grenzflächenaktive polyalkoxylierte Fettalkohole, die geeignet sind, einen hydrotropen Charakter zu gewährleisten, mit einem Gehalt zwischen 2,5 % und 7,5 % (Gew.-%) umfasst.

17. Verwendung nach Anspruch 16, wobei die wässerige Kupfer-Lösung ein Dritt-Lösungsmittel vom Alkohol- oder Polyol-Typ mit einem Gehalt zwischen 4 % und 7 % (Gew.-%) umfasst.

18. Verwendung nach einem der Ansprüche 10 bis 17, wobei die wässerige Kupfer-Lösung ein Färbemittel umfasst, das mit den Metallsalzen und dem Wasserstoffperoxid kompatibel ist.

19. Verwendung nach Anspruch 10, wobei die die wässerige Kupfer-Lösung umfasst (Gew.-%):
- Kupfersalze zwischen 0,1 % und 0,25 %,
- versalzte anionische grenzflächenaktive Verbindung zwischen 20 % und 30 %,
- grenzflächenaktive Stoffe vom Polyglucosid-Fettalkohol-Typ zwischen 2,5 % und 10 %,
- grenzflächenaktiver polyalkoxylierter Fettalkohol zwischen 2,5 % und 7,5 %,
- Dritt-Lösungsmittel vom Fettalkohol- oder Polyol-Typ zwischen 4 % und 7 %,
- Färbemittel q.s.,
- entmineralisiertes Wasser q.s. 100 %,
wobei die Summe der Bestandteile gleich 100 % ist.

20. Verwendung nach einem der Ansprüche 9 bis 19, wobei die wässerige Wasserstoffperoxid-Lösung, die dazu bestimmt ist, mit einer wässerigen Kupfer-Lösung gemischt zu werden, zur Herstellung der Zusammensetzung Wasserstoffperoxid-Stabilisatoren enthält, die aus Phosphonaten, Perboraten mit einem Gehalt zwischen 0,002 Gew.-% und 0,07 Gew.-% ausgewählt werden.

21. Verwendung nach einem der Ansprüche 9 bis 20, wobei die wässerige Kupfer-Lösung der wässerigen Wasserstoffperoxid-Lösung in 1/25 anzeigenden Anteilen zugesetzt wird.

22. Verwendung nach einem der Ansprüche 1 bis 21 zur Behandlung von Endoskopen.

23. Verwendung nach einem der Ansprüche 1 bis 22, wobei zuvor Schritte der Reinigung und/oder Desinfektion durchgeführt werden, um physikalisch und/oder enzymatisch einen Teil der CTA-Partikel oder Prionen zu eliminieren.

## Claims

1. The use of a composition for simultaneously disinfection and decontamination of objects or surfaces contaminated by prions and conventional transmissible agents (CTAs), such as in particular bacteriae, yeasts, viruses, mould spores, mycobacteriae, bacteriae spores, said composition comprising a solution of hydrogen peroxide and an aqueous solution of copper or a derivative thereof such as copper salts, wherein said composition further comprises at least one anionic salified surfactant compound inhibiting the decomposition of hydrogen peroxide in the presence of copper and wherein the concentration of hydrogen peroxide in the composition ranges between 5% and 9% by weight.

2. The use according to claim 1, wherein said composition comprises (% by weight):
- anhydrous copper salts between 0.005% and 0.01%,
- hydrogen peroxide between 5% and 9%,
- anionic salified surfactant compound between 0.5% and 1.5%,
- demineralised water q.s. 100%,
the sum of the components being equal to 100%.

3. The use according to claim 1 or 2, wherein said the at least one anionic salified surfactant compound is selected among the following group: dodecylbenzenesulfonate, dodecylsulfate, sodium dodecylethersulfate.

4. The use according to any one of claims 1 to 3, wherein said the at least one anionic salified surfactant compound is ultra-pure, of synthesis quality.

5. The use according to any one of claims 1 to 4, wherein the composition comprises surfactants such as especially selected polyglucoside fatty alcohols, in particular C4 alkyls, for solubilising copper salts.

6. The use according to claim 5, wherein said composition comprises polyalkoxylated fatty alcohol surfactants for assigning a hydrotropic character to the composition, in combination with a solubilising agent such as alcohols or polyols.

7. The use according to any one of claims 1 to 6, wherein said composition comprises a colouring agent compatible with metal salts and hydrogen peroxide.

8. The use according to any of the claims 1, 3 or 4, wherein said composition comprises (% by weight):
- copper salts between 0.005% and 0.01%,
- hydrogen peroxide between 5% and 9%,
- anionic salified surfactant compound between 0.5% and 1.5%,
- surfactants such as polyglucoside fatty alcohols between 0.1% and 0.4%,
- polyalkoxylated fatty alcohol surfactant between 0.1% and 0.3%,
- solubilising agent such as alcohols or polyols, between 0.1% and 0.3%,
- colouring agent q.s.,
- water, q.s. 100%,
the sum of the components being equal to 100%.

9. The use according to any one of claims 1 to 8, wherein said composition can be prepared by extemporaneously mixture of an aqueous copper solution and of an aqueous hydrogen peroxide solution.

10. The use according to claim 9, wherein said aqueous copper solution, intended for being mixed with an aqueous hydrogen peroxide solution, further comprises said at least one anionic salified surfactant compound inhibiting the decomposition of hydrogen peroxide in the presence of copper.

11. The use according to claim 10, wherein said anionic salified surfactant compound has a content ranging between 1% and 50% (by weight) in said aqueous copper solution.

12. The use according to claim 10 or 11, wherein said aqueous copper solution includes copper salt with a content ranging between 0.1% and 0.25% (by weight).

13. The use according to any one of claims 10 to 12, wherein said the at least one anionic salified surfactant compound of the aqueous copper solution is selected among the following the group: dodecylbenzenesulfonate, dodecylsulfate and sodium dodecylethersulfate.

14. The use according to any one of claims 10 to 13, wherein said the at least one anionic salified surfactant compound of the aqueous copper solution is ultra-pure, of synthesis quality.

15. The use according to any one of claims 10 to 14, wherein the aqueous copper solution comprises at least one surfactant such as polyglucoside fatty alcohols for solubilising copper salts, with a content ranging between 2.5% and 10% (% by weight).

16. The use according to any one of claims 10 to 15, wherein the aqueous copper solution comprises polyalkoxylated fatty alcohol surfactants for assigning a hydrotropic character, with a content ranging between 2.5% and 7.5% (% by weight).

17. The use according to claim 16, wherein said aqueous copper solution comprises a solubilising agent, such as alcohols or polyols with a content ranging between 4% and 7% (% by weight).

18. The use according to any one of claims 10 to 17, wherein said aqueous copper solution comprises a colouring agent compatible with metal salts and hydrogen peroxide.

19. The use according to claim 10, wherein said aqueous copper solution comprises (% by weight):
- copper salts between 0.1% and 0.25%,
- anionic salified surfactant compound between 20% and 30%,
- surfactants such as polyglucoside fatty alcohols between 2.5% and 10%,
- polyalkoxylated fatty alcohol surfactant between 2.5% and 7.5%,
- solubilising agent such as alcohols or polyols, between 4% and 7%,
- colouring agent q.s.,
- demineralised water, q.s. 100%,
the sum of the components being equal to 100%.

20. The use according to any one of claims 9 to 19, wherein said the aqueous hydrogen peroxide solution to be mixed with an aqueous copper solution, for the realisation of said composition contains hydrogen peroxide stabilising agents selected among phosphonates and perborates, with a content ranging between 0.002% and 0.07% by weight.

21. The use according to any one of claims 9 to 20, wherein said aqueous copper solution is added to the aqueous hydrogen peroxide solution in 1/25 indicative proportions.

22. The use according to any one of claims 1 to 21 for the treatment of endoscopes.

23. The use according to any one of claims 1 to 22, wherein steps of detergent stripping and/or disinfection are performed previously for eliminating a portion, physically and/or enzymatically, of CTA particles or prions.
